# EUROPEAN PATENT SPECIFICATION

(11) **EP 2 478 001 B1**
(45) Date of publication and mention of the grant of the patent: **25.04.2018**
(21) Application number: 10817616.5
(22) Date of filing: 28.07.2010
(51) Int. Cl.: C07K 5/12, C07K 5/10, A61K 38/12, A61K 38/07, A61P 25/30, A61P 25/04

(54) **ADVANTAGEOUS MU-OPIATE RECEPTOR PEPTIDE COMPOUNDS**
VORTEILHAFTE MU-OPIAT-REZEPTORPEPTIDVERBINDUNGEN
COMPOSÉS PEPTIDIQUES BÉNÉFIQUES DE RÉCEPTEUR AUX OPIACÉS MU

(30) Priority: 15.09.2009 US 559994
(43) Date of publication of application: 25.07.2012
(73) Proprietor: Cytogel Pharma, LLC, Darien, CT 06820 (US)
(72) Inventor: MAIONE, Theodore, E., Troy NY 12180 (US)
(74) Representative: Gill Jennings & Every LLP
(86) International application number: PCT/US2010/043482
(87) International publication number: WO 2011/034659

(56) References cited:
- WO-A1-2009/076672
- WO-A2-2011/146922
- US-A1- 2003 068 672
- US-A1- 2003 139 446
- US-A1- 2004 266 805
- US-B1- 6 303 578
- CARDILLO G ET AL: "Synthesis and binding activity of endomorphin-1 analogues containing beta-amino acids", BIOORGANIC & MEDICINAL CHEMISTRY LETTERS, PERGAMON, AMSTERDAM, NL, vol. 10, no. 24, 18 December 2000 (2000-12-18), pages 2755-2758, XP027377551, ISSN: 0960-894X [retrieved on 2000-12-18]
- AGNES, R. ET AL.: 'Structure-activity relationships of bifunctional peptides based on overlapping pharmacophores at opioid and cholecystokinin receptors' J MED CHEM vol. 49, no. 10, May 2006, pages 2868 - 2875, XP008160433
- NEUMEYER, J. ET AL.: 'New opioid designed multiple ligand from Dmt-Tic and morphinan pharmacophores' J MED CHEM vol. 49, no. 18, September 2006, pages 5640 - 5643, XP008160434

### SEQUENCE LISTING

<110> Cytogel, LLC.
   Maione, Theodore E.
<120> ADVANTAGEOUS MU-OPIATE RECEPTOR PEPTIDE COMPOUNDS
<130> FXS00115EP
<160> 26
<170> PatentIn version 3.5
<210> 1
   <211> 4
   <212> PRT
   <213> Homo sapiens
<220>
   <221> MISC_FEATURE
   <222> (4)..(4)
   <223> Amidated Phenylalanine
<400> 1
   Tyr Pro Trp Phe
   1
<210> 2
   <211> 4
   <212> PRT
   <213> Homo sapiens
<220>
   <221> MISC_FEATURE
   <222> (4)..(4)
   <223> Amidated Phenylalanine
<400> 2
   Tyr Pro Phe Phe
   1
<210> 3
   <211> 4
   <212> PRT
   <213> Artificial Sequence
<220>
   <223> Synthetic mu-opiate receptor peptides
<220>
   <221> MISC_FEATURE
   <222> (4)..(4)
   <223> Hydroxylated Phenylalanine
<400> 3
   Tyr Pro Trp Phe
   1
<210> 4
   <211> 4
   <212> PRT
   <213> Artificial Sequence
<220>
   <223> Synthetic mu-opiate receptor peptides
<220>
   <221> MISC_FEATURE
   <222> (4)..(4)
   <223> Hydroxylated Phenylalanine
<400> 4
   Tyr Pro Phe Phe
   1
<210> 5
   <211> 4
   <212> PRT
   <213> Artificial Sequence
<220>
   <223> Synthetic mu-opiate receptor peptides
<220>
   <221> MISC_FEATURE
   <222> (4)..(4)
   <223> Amidated Phenylalanine
<400> 5
   Tyr Pro Trp Phe
   1
<210> 6
   <211> 4
   <212> PRT
   <213> Artificial Sequence
<220>
   <223> Synthetic mu-opiate receptor peptides
<400> 6
   Tyr Pro Phe Phe
   1
<210> 7
   <211> 4
   <212> PRT
   <213> Artificial Sequence
<220>
   <223> Synthetic mu-opiate receptor peptides
<220>
   <221> MISC_FEATURE
   <222> (3)..(3)
   <223> Nitrosylated Tryptophan
<220>
   <221> MISC_FEATURE
   <222> (4)..(4)
   <223> Amidated Phenylalanine
<400> 7
   Tyr Pro Trp Phe
   1
<210> 8
   <211> 4
   <212> PRT
   <213> Artificial Sequence
<220>
   <223> Synthetic mu-opiate receptor peptides
<220>
   <221> MISC_FEATURE
   <222> (3)..(3)
   <223> Nitrosylated Phenylalanine
<220>
   <221> MISC_FEATURE
   <222> (4)..(4)
   <223> Amidated Phenylalanine
<400> 8
   Tyr Pro Phe Phe
   1
<210> 9
   <211> 4
   <212> PRT
   <213> Artificial Sequence
<220>
   <223> Synthetic mu-opiate receptor peptides
<220>
   <221> MISC_FEATURE
   <222> (3)..(3)
   <223> Alkylated Phenylalanine
<220>
   <221> MISC_FEATURE
   <222> (4)..(4)
   <223> Amidated Phenylalanine
<400> 9
   Tyr Pro Phe Phe
   1
<210> 10
   <211> 4
   <212> PRT
   <213> Artificial Sequence
<220>
   <223> Synthetic mu-opiate receptor peptides
<220>
   <221> MISC_FEATURE
   <222> (3)..(3)
   <223> Alkylated Phenylalanine
<220>
   <221> MISC_FEATURE
   <222> (4)..(4)
   <223> Amidated Phenylalanine
<400> 10
   Tyr Pro Phe Phe
   1
<210> 11
   <211> 4
   <212> PRT
   <213> Artificial Sequence
<220>
   <223> Synthetic mu-opiate receptor peptides
<220>
   <221> MISC_FEATURE
   <222> (3)..(3)
   <223> Alkylated Phenylalanine
<220>
   <221> MISC_FEATURE
   <222> (4)..(4)
   <223> Amidated Phenylalanine
<400> 11
   Tyr Pro Phe Phe
   1
<210> 12
   <211> 4
   <212> PRT
   <213> Artificial Sequence
<220>
   <223> Synthetic mu-opiate receptor peptides
<220>
   <221> MISC_FEATURE
   <222> (3)..(3)
   <223> Alkylated Phenylalanine
<220>
   <221> MISC_FEATURE
   <222> (4)..(4)
   <223> Amidated Phenylalanine
<400> 12
   Tyr Pro Phe Phe
   1
<210> 13
   <211> 4
   <212> PRT
   <213> Artificial Sequence
<220>
   <223> Synthetic mu-opiate receptor peptides
<220>
   <221> MISC_FEATURE
   <222> (2)..(4)
   <223> Cyclic Peptide
<400> 13
   Tyr Lys Trp Phe
   1
<210> 14
   <211> 4
   <212> PRT
   <213> Artificial Sequence
<220>
   <223> Synthetic mu-opiate receptor peptides
<220>
   <221> MISC_FEATURE
   <222> (2)..(4)
   <223> Cyclic Peptide
<400> 14
   Tyr Lys Phe Phe
   1
<210> 15
   <211> 4
   <212> PRT
   <213> Artificial Sequence
<220>
   <223> Synthetic mu-opiate receptor peptides
<220>
   <221> MISC_FEATURE
   <222> (2)..(4)
   <223> Cyclic Peptide
<220>
   <221> MISC_FEATURE
   <222> (2)..(2)
   <223> Ornathine
<400> 15
   Tyr Xaa Trp Phe
   1
<210> 16
   <211> 4
   <212> PRT
   <213> Artificial Sequence
<220>
   <223> Synthetic mu-opiate receptor peptides
<220>
   <221> MISC_FEATURE
   <222> (2)..(4)
   <223> Cyclic Peptide
<220>
   <221> MISC_FEATURE
   <222> (2)..(2)
   <223> Ornathine
<400> 16
   Tyr Xaa Phe Phe
   1
<210> 17
   <211> 4
   <212> PRT
   <213> Artificial Sequence
<220>
   <223> Synthetic mu-opiate receptor peptides
<220>
   <221> MISC_FEATURE
   <222> (2)..(4)
   <223> Cyclic Peptide
<220>
   <221> MISC_FEATURE
   <222> (3)..(3)
   <223> Nitrosylated Tryptophan
<400> 17
   Tyr Lys Trp Phe
   1
<210> 18
   <211> 4
   <212> PRT
   <213> Artificial Sequence
<220>
   <223> Synthetic mu-opiate receptor peptides
<220>
   <221> MISC_FEATURE
   <222> (2)..(4)
   <223> Cyclic Peptide
<220>
   <221> MISC_FEATURE
   <222> (3)..(3)
   <223> Nitrosylated Phenylalanine
<400> 18
   Tyr Lys Phe Phe
   1
<210> 19
   <211> 4
   <212> PRT
   <213> Artificial Sequence
<220>
   <223> Synthetic mu-opiate receptor peptides
<220>
   <221> MISC_FEATURE
   <222> (2)..(4)
   <223> Cyclic Peptide
<220>
   <221> MISC_FEATURE
   <222> (2)..(2)
   <223> Ornathine
<220>
   <221> MISC_FEATURE
   <222> (3)..(3)
   <223> Nitrosylated Tryptophan
<400> 19
   Tyr Xaa Trp Phe
   1
<210> 20
   <211> 4
   <212> PRT
   <213> Artificial Sequence
<220>
   <223> Synthetic mu-opiate receptor peptides
<220>
   <221> MISC_FEATURE
   <222> (2)..(4)
   <223> Cyclic Peptide
<220>
   <221> MISC_FEATURE
   <222> (2)..(2)
   <223> Ornathine
<220>
   <221> MISC_FEATURE
   <222> (3)..(3)
   <223> Nitrosylated Phenylalanine
<400> 20
   Tyr Xaa Phe Phe
   1
<210> 21
   <211> 4
   <212> PRT
   <213> Artificial Sequence
<220>
   <223> Synthetic mu-opiate receptor peptides
<220>
   <221> MISC_FEATURE
   <222> (2)..(4)
   <223> Cyclic Peptide
<220>
   <221> MISC_FEATURE
   <222> (3)..(3)
   <223> Alkylated Phenylalanine
<400> 21
   Tyr Lys Phe Phe
   1
<210> 22
   <211> 4
   <212> PRT
   <213> Artificial Sequence
<220>
   <223> Synthetic mu-opiate receptor peptides
<220>
   <221> MISC_FEATURE
   <222> (2)..(4)
   <223> Cyclic Peptide
<220>
   <221> MISC_FEATURE
   <222> (2)..(2)
   <223> Ornathine
<220>
   <221> MISC_FEATURE
   <222> (3)..(3)
   <223> Alkylated Phenylalanine
<400> 22
   Tyr Xaa Phe Phe
   1
<210> 23
   <211> 4
   <212> PRT
   <213> Artificial Sequence
<220>
   <223> Synthetic mu-opiate receptor peptides
<220>
   <221> MISC_FEATURE
   <222> (2)..(4)
   <223> Cyclic Peptide
<220>
   <221> MISC_FEATURE
   <222> (3)..(3)
   <223> Alkylated Phenylalanine
<400> 23
   Tyr Lys Phe Phe
   1
<210> 24
   <211> 4
   <212> PRT
   <213> Artificial Sequence
<220>
   <223> Synthetic mu-opiate receptor peptides
<220>
   <221> MISC_FEATURE
   <222> (2)..(4)
   <223> Cyclic Peptide
<220>
   <221> MISC_FEATURE
   <222> (2)..(2)
   <223> Ornathine
<220>
   <221> MISC_FEATURE
   <222> (3)..(3)
   <223> Alkylated Phenylalanine
<400> 24
   Tyr Xaa Phe Phe
   1
<210> 25
   <211> 4
   <212> PRT
   <213> Artificial Sequence
<220>
   <223> Synthetic mu-opiate receptor peptides
<220>
   <221> MISC_FEATURE
   <222> (2)..(4)
   <223> Cyclic Peptide
<220>
   <221> MISC_FEATURE
   <222> (3)..(3)
   <223> Alkylated Phenylalanine
<400> 25
   Tyr Lys Phe Phe
   1
<210> 26
   <211> 4
   <212> PRT
   <213> Artificial Sequence
<220>
   <223> Synthetic mu-opiate receptor peptides
<220>
   <221> MISC_FEATURE
   <222> (2)..(4)
   <223> Cyclic Peptide
<220>
   <221> MISC_FEATURE
   <222> (3)..(3)
   <223> Alkylated Phenylalanine
<400> 26
   Tyr Lys Phe Phe
   1

## Description

### BACKGROUND OF INVENTION

### Field of the Invention

This invention relates to a salt of a peptide that binds with high affinity and selectivity to the mu (morphine) opiate receptor; pharmaceutical preparations containing an effective amount of the compound and therapy for drug dependence containing an effective amount of the peptide compound.

### Description of the Related Art

Many peptides have been found that exhibit opiate-like activity by binding to opiate receptors. Three different types of opiate receptors have been found: delta (δ), kappa (κ) and mu (µ). The major putative function for opiates is their role in alleviating pain. Other areas where opiates are well-suited for use in treatment are conditions relating to gastrointestinal disorders, schizophrenia, obesity, blood pressure, convulsions, and seizures. Although the δ and κ receptors may also mediate analgesia, activation of µ receptors is the primary and most effective means of inducing analgesia, and is the primary mechanism by which morphine acts.

Because morphine and other compounds with clinical usefulness act primarily at the µ receptor, pharmaceutical compositions having peptides with high affinity and selectivity for this site are of considerable importance. It would be desirable to produce these peptide compositions in a simple, efficient, and economical manner.

### BRIEF SUMMARY

The subject invention provides an advantageous new salt of a mu-opiate receptor peptide. This compound has been found to have excellent activity.

Specifically exemplified herein is a trifluoroacetate salt of a mu-opiate receptor peptide.

The subject invention further provides pharmaceutical compositions comprising the advantageous peptide compound.

The subject invention further provides a therapeutic method that utilizes the salt and compositions described herein.

### BRIEF DESCRIPTION OF SEQUENCES

SEQ ID NO:1 is a peptide disclosed herein.
SEQ ID NO:2 is a peptide disclosed herein.
SEQ ID NO:3 is a peptide disclosed herein.
SEQ ID NO:4 is a peptide disclosed herein.
SEQ ID NO:5 is a peptide. disclosed herein
SEQ ID NO:6 is a peptide disclosed herein.
SEQ ID NO:7 is a peptide disclosed herein.
SEQ ID NO:8 is a peptide disclosed herein.
SEQ ID NO:9 is a peptide disclosed herein.
SEQ ID NO:10 is a peptide disclosed herein.
SEQ ID NO:11 is a peptide disclosed herein.
SEQ ID NO: 12 is a peptide disclosed herein.
SEQ ID NO:13 is a peptide according to the invention.
SEQ ID NOs: 14-26 are additional peptides disclosed herein.

### DETAILED DISCLOSURE

The subject invention provides an advantageous salt of a peptide that binds to the mu (morphine) opiate receptor with high affinity, selectivity and potency.

Specifically exemplified herein is a trifluoroacetate (TFA) salt of a mu-opiate receptor peptide.

Advantageously, the compound of the subject invention has excellent properties in terms of its activity.

This invention also provides pharmaceutical preparations containing an effective amount of the peptide salt. The subject invention further provides therapy for drug dependence wherein the therapy involves administering, to a patient in need of such treatment, a composition containing an effective amount of the peptide compound of the subject invention.

### Peptides

The peptide that can be used according to the subject invention is H-Tyr-c-[D-Lys-Trp-Phe] (SEQ ID NO:13).
Some other peptides disclosed herein are:
H-Tyr-Pro-Trp-Phe-NH₂ (SEQ ID NO:1)
H-Tyr-Pro-Phe-Phe-NH₂ (SEQ ID NO:2)
H-Tyr-Pro-Trp-Phe-OH (SEQ ID NO:3)
H-Tyr-Pro-Phe-Phe-OH (SEQ ID NO:4)
H-Tyr-Pro-Trp-D-Phe-NH₂ (SEQ ID NO:5)
H-Tyr-Pro-Phe-D-Phe-NH₂ (SEQ ID NO:6)
H-Tyr-Pro-Trp-pNO₂ -Phe-NH₂ (SEQ ID NO:7)
H-Tyr-Pro-Phe-pNO₂ -Phe-NH₂ (SEQ ID NO:8)
H-Tyr-Pro-N-Me-Phe-Phe-NH₂ (SEQ ID NO:9)
H-Tyr-Pro-N-Et-Phe-Phe-NH₂ (SEQ ID NO:10)
H-Tyr-Pro-N-Me-Phe-D-Phe-NH₂ (SEQ ID NO:11)
H-Tyr-Pro-N-Et-Phe-D-Phe-NH₂ (SEQ ID NO:12)
H-Tyr-c-[D-Lys-Phe-Phe] (SEQ ID NO:14)
H-Tyr-c-[D-Orn-Trp-Phe] (SEQ ID NO:15)
H-Tyr-c-[D-Orn-Phe-Phe] (SEQ ID NO:16)
H-Tyr-c-[D-Lys-Trp-pNO₂ -Phe] (SEQ ID NO:17)
H-Tyr-c-[D-Lys-Phe-pNO₂ -Phe] (SEQ ID NO:18)
H-Tyr-c-[D-Orn-Trp-pNO₂ -Phe] (SEQ ID NO:19)
H-Tyr-c-[D-Orn-Phe-pNO₂ -Phe] (SEQ ID NO:20)
H-Tyr-c-[D-Lys-N-Me-Phe-Phe] (SEQ ID NO:21)
H-Tyr-c-[D-Orn-N-Me-Phe-Phe] (SEQ ID NO:22)
H-Tyr-c-[D-Lys-N-Et-Phe-Phe] (SEQ ID NO:23)
H-Tyr-c-[D-Orn-N-Et-Phe-Phe] (SEQ ID NO:24)
H-Tyr-c-[D-Lys-N-Me-Phe-D-Phe] (SEQ ID NO:25)
H-Tyr-c-[D-Lys-N-Et-Phe-D-Phe] (SEQ ID NO:26)

The last fourteen peptides listed are cyclic peptides whose linear primary amino acid sequences are given in SEQ ID NO:13 through SEQ ID NO:26.

The peptide of SEQ ID NO:1 is highly selective and very potent for the .mu.opiate receptor, with over 4000-fold weaker binding to delta receptors and over 15,000-fold weaker binding to kappa receptors, reducing the chances of side-effects. WO2009/076672 A1 discloses peptides having SEQ ID NOs: 1-26 and various salts thereof, as well as their use in the treatment of a condition modulated by a mu-opiate receptor. Trifluoro acetate salts of peptides of SEQ ID NOs: 13-26 are not disclosed therein. The document discloses the free base and acetate salt of the peptide of SEQ ID NO: 13.

Cardillo et al., Bioorg. Med. Chem. Lett. 10 (2000) 2755-2758 discloses a trifluoro acetate salt of SEQ ID: NO:1.

The peptide of this invention may be prepared by conventional solution-phase (Bodansky, M., Peptide Chemistry: A Practical Textbook, 2nd Edition, Springer-Verlag, New York (1993) or solid phase (Stewart, J.M.; Young, J.D. Solid Phase Peptide Synthesis, 2nd edition, Pierce Chemical Company, 1984) methods with the use of proper protecting groups and coupling agents. A suitable deprotection method may then be employed to remove specified or all of the protecting groups, including splitting off the resin if solid phase synthesis is applied.

Cyclization of the linear peptide can be performed by, for example, substitution of an appropriate diamino carboxylic acid for Pro in position 2 in the peptides through ring closure of the 2-position side chain amino and the C-terminal carboxylic functional groups. The cyclization reactions can be performed with the diphenylphosphoryl azide method (Schmidt, R., Neuhert, K., Int. J. Pept. Protein Res. 37:502-507, 1991).

Peptides synthesized with solid phase synthesis can be split off the resin with liquid hydrogen fluoride (HF) in the presence of the proper antioxidant and scavenger.

The amount of the reactants utilized in the reactions, as well as the conditions required to facilitate the reactions and encourage efficient completion may vary widely depending on variations in reaction conditions and the nature of the reactants.

The desired products may be isolated from the reaction mixture by crystallization, electrophoresis, extraction, chromatography, or other means. However, a preferred method of isolation is HPLC. All of the crude peptides can be purified with preparative HPLC, and the purity of the peptides may be checked with analytical HPLC. Purities greater than 95% of the synthesized compounds using HPLC have been obtained.

SEQ ID NO:13 (cyclic endomorphin-1 peptide) has the following structure: 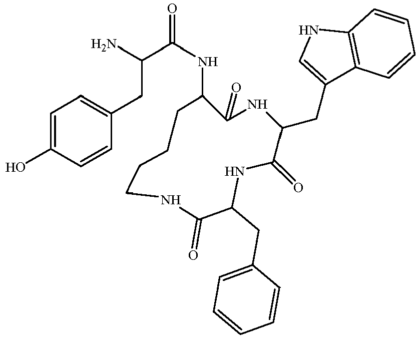 Cyt 1010
C₃₅H₄₀N₆O₅
Mol. Wt:624.73
C, 67.29; H, 6.45; N, 13.45: O, 12.81

### Pharmaceutical Compositions

The present invention also provides pharmaceutical preparations that contain a pharmaceutically effective amount of the peptide salt of this invention and a pharmaceutically acceptable carrier or adjuvant. The carrier may be an organic or inorganic carrier that is suitable for external, enteral or parenteral applications.

The peptide salt of the present invention may be compounded, for example, with the usual non-toxic, pharmaceutically acceptable carriers for tablets, pellets, capsules, liposomes, suppositories, intranasal sprays, solutions, emulsions, suspensions, aerosols, targeted chemical delivery systems (Prokai-Tatrai, K.; Prokai, L; Bodor, N., J. Med. Chem. 39:4775-4782, 1991), and any other form suitable for use. The carriers which can be used are water, glucose, lactose, gum acacia, gelatin, mannitol, starch paste, magnesium trisilicate, talc, corn starch, keratin, colloidal silica, potato starch, urea and other carriers suitable for use in manufacturing preparations, in solid, semisolid, liquid or aerosol form, and in addition auxiliary, stabilizing, thickening and coloring agents and perfumes may be used.

### Therapeutic Methods

The present invention also provides therapy for drug dependence in patients, such as mammals, including humans, which comprise administering to the patient an effective amount of the salt of this invention. For applying the peptide salt of the present invention to human, it is preferable to administer it by parenteral or enteral administration.

The peptide salt of the subject invention can also be used to provide antiinflammatory treatments.

The dosage of effective amount of the peptide varies from and also depends upon the age and condition of each individual patient to be treated. However, suitable unit dosages may be between about 0.01 to about 100 mg. For example, a unit dose may be from between about 0.2 mg to about 50 mg. Such a unit dose may be administered more than once a day, e.g. two or three times a day.

Following is an example which illustrates aspects of the invention.

### EXAMPLE 1 - ACTIVITY

The assay was the standard rat tail flick assay. Test agents were administered intravenously as suspensions in 20 % PEG.

| CYT-1010 Salt form | IV Dose (mg/kg) | Activity % MPE Ave |
|---|---|---|
| Vehicle | | 1.0 |
| Acetate (reference) | 2 | 50.0 |
| | 4 | 83.3 |
| TFA (according to the invention) | 4 | 25.4 |
| | 8 | 71.2 |
| Free Base (reference) | 2 | 77.7 |
| | 4 | 100.0 |
| HCL (reference) | 4 | 0.0 |
| Aspartate (reference) | 2 | 7.4 |
| Lactate (reference) | 2 | 1.4 |

## Claims

1. A peptide compound wherein the peptide is SEQ ID NO: 13 and the compound is a trifluoro acetate salt.

2. A pharmaceutical composition comprising the trifluoro acetate salt of SEQ ID NO: 13.

3. A trifluoroacetate salt of a peptide having SEQ ID NO: 13 for use in the treatment of drug dependence in a patient.

4. The trifluoroacetate salt according to claim 3, wherein the peptide is SEQ ID NO:13.

## Patentansprüche

1. Peptidverbindung, wobei das Peptid SEQ.-ID Nr. 13 ist und die Verbindung ein Trifluoracetatsalz ist.

2. Pharmazeutische Zusammensetzung, die das Trifluoracetatsalz von SEQ.-ID Nr. 13 umfasst.

3. Trifluoracetatsalz eines Peptid, das SEQ.-ID Nr. 13 aufweist, für die Verwendung bei der Behandlung von Drogenabhängigkeit bei einem Patienten.

4. Trifluoracetatsalz nach Anspruch 3, wobei das Peptid SEQ.-ID Nr. 13 ist.

## Revendications

1. Composé peptidique, où le peptide est la séquence SEQ ID N° : 13 et le composé est un sel de type trifluoroacétate.

2. Composition pharmaceutique comprenant le sel de type trifluoroacétate de la séquence SEQ ID N° : 13.

3. Sel de type trifluoroacétate d'un peptide ayant la séquence SEQ ID N° : 13 pour une utilisation dans le traitement de la pharmacodépendance chez un patient.

4. Sel de type trifluoroacétate selon la revendication 3, où le peptide est la séquence SEQ ID N° : 13.
